# EUROPEAN PATENT APPLICATION

(11) **EP 1 550 870 A1**
(43) Date of publication of application: **06.07.2005**
(21) Application number: 03733182.4
(22) Date of filing: 29.05.2003
(51) Int. Cl.: G01N 33/53, G01N 37/00, C12N 15/00, C12M 1/00

(54) **METHOD FOR DETECTING BIOCHEMICAL REAGIN BIOCHIP**

(30) Priority: 31.05.2002 JP 2002159350
(71) Applicant: Sumitomo Precision Products Co., Ltd., Amagasaki-shi, Hyogo 660-0891 (JP)
(72) Inventor: YABUBAYASHI, Tadaaki, SUMITOMO PRECISION PROD. CO., Amagasaki-shi, Hyogo 660-0891 (JP); TANAKA, Masazumi, c/o SUMITOMO PRECISION PROD. CO., Amagasaki-shi, Hyogo 660-0891 (JP)
(74) Representative: Peel, James Peter
(86) International application number: PCT/JP2003/006791
(87) International publication number: WO 2003/102582

(57) **Abstract**

A detecting method with an improved accuracy of detection of an object specimen, finally achieved by simplifying the step of detecting a biochemical reactant by means of a biochip with good reproducibility and enhancing the accuracy of the operation and its effect at the hybridization and label modification steps without requiring, the measuring operator to have an excessively high level of skill. A probe nucleic acid is made to have a three-dimensional structure by adopting a loop structure in such a way that the free end not fixed to the surface of a substrate or a portion where a label is modifiable is located on an electrode of a biochip or near the surface of the electrode on the substrate side, or that the essential portion complementarily connected to the biochemical specimen is located on the substrate side. As a result the probe nucleic acid has a property that it can be basically hybridized with only the object specimen. When the probe nucleic acid is hybridized with the sample specimen, the loop structure is destroyed if the object specimen is present, and the label of only the hybridized probe can be modified. The disturbance noise is reduced because the hybridization and the label modification are selectively carried out at the hybridization and label modification steps, greatly improving the accuracy of detection of the object specimen.

## Description

### TECHNICAL FIELD

The present invention relates to a method for detecting a biochemical reactant with improved accuracy of hybridization and label modification themselves which are used as electrical, electromagnetic, electro-optical or electro-magneto-optical changes to achieve highly precise detection of a biochemical specimen, wherein in an improvement of a method for detecting a biochemical specimen using a biochip, a probe nucleic acid arrayed on a substrate are made to have a loop structure wherein for example the free end is located on the substrate side, so that hybridization only occurs when the target DNA or RNA is present, and the aforementioned loop structure is destroyed and stretched out so that only the hybridized target complex (biochemical reactant) can be modified with any label.

### BACKGROUND ART

The basic principle of genetic detection makes use of the fact that DNA forms complementary double helix structures. Because A (adenine) is paired with T (thymine) and C (cytosine) with G (guanine), for example a gene having the DNA sequence A G G T T A C (5'-- > 3') can be detected by preparing DNA having the sequence T C C A A T G (3'-->5') as a probe, so that if the target gene is present in the sampling specimen genes the AGGTTAC sequence will bind to the probe DNA by DNA hybridization, forming a double helix structure which can be detected in order to easily select the target DNA.

Known methods of detecting double helix DNA include fluorescence methods in which the specimen DNA (sampling gene DNA) is modified with a fluorescent label, a hybridization operation is performed with the aforementioned DNA and probe DNA, and DNA exhibiting a double helix structure is detected by detecting the fluorescent signal.

In addition to fluorescent dyes themselves, a variety of methods and substances have been proposed for the fluorescence-emitting labels for fluorescent labeling, such as chromosomes directly dyed with fluorescent dye, ionic fluorescent substances used to modify sugar chains extracted from glycoproteins or glycolipids, and proteins, nucleic acids, enzymes, cells or the like tagged with fluorescent dyes.

In addition to the aforementioned DNA, a wide variety of structures are supposed as the biochemical specimen which is the object of detection, including RNA and nucleic acid-like structures. In order to efficiently detect these biochemical specimens, it is necessary to array DNA or an RNA structure or PNA structure with a specific nucleotide sequence which functions as a probe on a specific glass or other substrate to form a chip, perform a hybridization operation with the biochemical specimen using this biochip as the basic unit, and reliably detect target complexes (biochemical reactants) of the biochemical specimen and the probe on completion of hybridization.

In methods of hybridizing with the aforementioned biochip and detecting hybridized DNA using a fluorescent method in which the specimen is fluorescent labeled, problems have arisen including complexity of the modifying operation with the fluorescent label, differences in efficiency of the aforementioned hybridization and label modification depending on the skill of the operator, extinction of the fluorescent dye under various conditions and diminished detection precision due to increases in background noise caused by adsorption of unreacted substances.

### DISCLOSURE OF THE INVENTION

In light of the aforementioned problems with methods of detecting a biochemical specimen using a biochip, it is an object of the present invention to provide a method for detecting a biochemical reactant and a biochip wherein improved accuracy of detection of the object specimen is finally achieved by simplifying the detection step with good reproducibility and enhancing the accuracy of the operation and its effects at the hybridization steps without requiring the measuring operator to have an excessively high level of skill.

In an effort to improve the accuracy of detection of a biochemical specimen using a biochip, the inventors discovered after exhaustive research into methods of hybridizing a biochemical specimen with a probe on a biochip having an array of a plurality of probes on a glass or other substrate and methods of label modification such as fluorescent labeling of a probe that problems exist including:
1) When a probe which has formed a double helix structure on completion of hybridization is modified with a label, the un-hybridized probe is also modified so that detection by means of the label is impossible; conventionally, the specimen is modified with a label in advance and the target specimen is detected by detecting the label of the hybridized specimen, but non-specific adsorption occurs during hybridization due to differences in condition settings and the skill of the operator, resulting in fluctuating or diminished accuracy of detection;
2) When the probe is RNA or a nucleic acid-like structure, binding with the biochemical specimen is stronger than when the probe is a DNA structure, and because several probes are arrayed on a substrate there is a high likelihood of adsorption or hybridization of non-targets, increasing the noise and diminishing the accuracy of detection of the target biochemical specimen,
   and realized that to solve these problems a new basic structure must be provided which fundamentally improves the accuracy of detection on a biochip with an array of a plurality of probes.

The inventors therefore performed various studies aimed at a new basic structure capable of improving the accuracy of detection on a biochip having a plurality of probes arrayed on a substrate, or in other words a configuration in which probes on a substrate can hybridize only with the target biochemical specimen, and realizing that accuracy of detection can be fundamentally improved by label modification of the biochemical reactant after hybridization, and focusing on improving the original accuracy of detection with a configuration which does not allow easy hybridization but allows hybridization only with the target biochemical specimen, they performed exhaustive research into structures and configurations of the probe itself which allow accuracy of detection to be improved.

As a result, the inventors realized that if a hybridized probe alone is stretched out further than the un-hybridized probes, only that tip will be modifiable with a label, and that the principal part of the probe which binds complementarily with the biochemical specimen cannot hybridize easily if it is buried within the arrayed probe but will hybridize selectively and reliably only with the biochemical specimen, and after research into configurations capable of giving the biochip a three-dimensional structure in order to achieve this and improve the accuracy of detection they discovered that by giving the probe arrayed on the substrate a loop structure so that the free end not fixed to the surface of the substrate or the part which is modifiable with a label is located on the substrate side, or so that the principal part which binds complementarily with the biochemical specimen is located on the substrate side on or near the surface of the substrate, it is possible to achieve the aforementioned selective hybridization and fundamentally improve the accuracy of detection.

Moreover, the inventors also discovered that with the configuration of a biochip having probes with this loop structure arrayed on a substrate, rather than hybridization using a biochemical specimen modified in advance with a label as described above, it becomes possible to reverse the conventional method and label modify the tips or other necessary sites of only hybridized probes or in other words probes the loop structures of which have been destroyed following hybridization with the biochemical specimen, thus improving accuracy during hybridization and label modification and in each step so that the accuracy of detection of the target specimen is dramatically improved and the operation of detecting a biochemical specimen is relatively easy because operator skill is fundamentally not required in each step in order to improve accuracy of detection.

In addition, the inventors perfected the present invention upon discovering that with the configuration of a biochip of the present invention, because selective label modification is possible only with the hybridized probe and specimen, any particles including conventional fluorescent labels and fluorescent dyes as well as metal particles including Si, magnetic particles, ceramic particles, chemical colorants, semiconductors and the like can be adopted for labeling, that an additional label modification can be added targeting an initially attached label and that multiple labels can be formed, so that a variety of label detection methods can be adopted according to the type of label used or in other words methods can be adopted which detect or recognize the presence or absence of a label on a hybridized biochemical reactant as electrical, magnetic or optical changes, and that the best labels and detection methods can be adopted according to the properties of the biochemical specimen to be detected.

Moreover, the inventors perfected the present invention when they discovered that by label modifying probes provided with this loop structure in advance, and performing the aforementioned electrical, magnetic or optical measurements or a combination of such measurements before the hybridization operation, it is possible to quantitatively assess the condition of probes arrayed on the electrodes of a biochip or in other words the conditions of the probes on each electrode before the various operations, so that when a label modification is applied to a biochemical specimen or probe nucleic acid which has formed a double chain following the hybridization operation, or to both, and these labels are detected in order to evaluate the presence or absence of complexes forming double chains, the correlations and evaluations can be made more accurate because the conditions of the probes on each electrode have been quantitatively assessed in advance, and that by making the label used to modify the probe in advance and the label used to modify after hybridization of different types or by using the same type but making morphological modifications to the dimensions, color or the like or varying the label detection method or adopting multiple techniques, it is possible to more accurately correct the detection results according to the condition of the probe (amount of probe on the electrode).

The present invention is a method for detecting a biochemical reactant having a step of hybridizing a biochemical specimen with a probe nucleic acid on a biochip having a probe nucleic acid forming a loop structure and arrayed on one or two or more electrodes provided on a surface of a substrate or substrate analog or a probe nucleic acid of the aforementioned configuration with a label modification added in advance, and a step of detecting/discriminating a complex of the probe nucleic acid forming a double chain with the biochemical specimen by means of at least one of electrical, magnetic or optical changes on the surface of the biochip.

Moreover, the present invention is a method for detecting a biochemical reactant having a step of hybridizing a biochemical specimen with a probe nucleic acid on a having a probe nucleic acid forming a loop structure and arrayed on one or two or more electrodes provided on a surface of a substrate or substrate analog or a probe nucleic acid of the aforementioned configuration with a label modification added in advance, a step of modifying with a label either during or after hybridization one or both of the biochemical specimen and the probe nucleic acid forming a double chain, and a step of detecting/discriminating a complex of the probe nucleic acid forming a double chain with the biochemical specimen by means of at least one of electrical, magnetic or optical changes on the surface of the biochip.

In addition, the present invention is a method for detecting a biochemical reactant having a step of hybridizing a biochemical specimen modified in advance with a label with a probe nucleic acid on a biochip having a probe nucleic acid forming a loop structure and arrayed on one or two or more electrodes provided on a surface of a substrate or substrate analog or a probe nucleic acid of the aforementioned configuration with a label modification added in advance, and a step of detecting/discriminating a complex of the probe nucleic acid forming a double chain with the biochemical specimen by means of at least one of electrical, magnetic or optical changes on the surface of the biochip.

Furthermore, the present invention is a method for detecting a biochemical reactant having a step of hybridizing a biochemical specimen modified in advance with a label with a probe nucleic acid on a biochip having a probe nucleic acid forming a loop structure and arrayed on one or two or more electrodes provided on a surface of a substrate or substrate analog or a probe nucleic acid of the aforementioned configuration with a label modification added in advance, a step of modifying with a label either during or after hybridization one or both of the biochemical specimen and the probe nucleic acid forming a double chain, and a step of detecting/discriminating a complex of the probe nucleic acid forming a double chain with the biochemical specimen by means of at least one of electrical, magnetic or optical changes on the surface of the biochip.

Moreover, in the aforementioned method for detecting a biochemical reactant the present invention provides altogether
-- in the detection/discrimination step, a method of comparing the measurement results for the biochip following each step against the standard of measurement results performed to assess at least one of electrical, magnetic and optical changes to the surface of the biochip before the hybridization operation,
-- in the detection/discrimination step, a method of performing measurements to assess at least one of electrical, magnetic and optical changes to the surface of the biochip before and after the hybridization operations or also before and after the label modification operation, and comparing these results,
-- in the detection/discrimination step, a method of performing measurements before the hybridization operation to assess at least one of electrical, magnetic and optical changes to the surface of a biochip having a plurality of electrodes, calculating the relative amounts of probe nucleic acids on each electrode in advance and using them as a correction reference for measurement values after each step,
-- a method wherein the method of modifying a probe nucleic acid or biochemical specimen with a label is a multi-stage method of two or three or more stages in which a second label is added targeting a previous attached first label,
-- a method wherein the method of modifying either or both of a probe nucleic acid and a biochemical specimen with a label is a multi-stage method of two or three or more stages in which modification with a first label is followed by modification with a second label targeting the first label,
-- a method wherein the labels are selected from fine metal particles (including Si), magnetic particles, ceramic fine particles, fluorescent labels, fluorescent dyes, dyes, chemical colorants and semiconductors,
-- a method of detecting/discriminating electrical changes on the surface of a biochip which is a method of detecting/discriminating at least one of changes in current values, voltage values or resistance values on a biochip or electrode or changes in capacitance on the surface of a biochip,
-- a method wherein the method of detecting/discriminating as electrical and magnetic changes on the surface of a biochip has a step of detecting/discriminating at least one of changes in current values, voltage values or resistance values on a biochip or electrode or changes in capacitance on the surface of a biochip and a step of magnetically detecting/discriminating a signal such as magnetism for example from a complex which forms a double chain or from a label modifying one or both of the probe nucleic acid or biochemical specimen of the complex, or from the complex and the aforementioned label,
-- a method wherein the method of detecting/discriminating as electrical and optical changes on the surface of a biochip has a step of detecting/discriminating at least one of changes in current values, voltage values or resistance values on a biochip or electrode or changes in capacitance on the surface of a biochip and a step of optically detecting/discriminating a signal such as light for example from a complex which forms a double chain, or from a label modifying one or both of the probe nucleic acid or biochemical specimen of that complex, or from the complex and the aforementioned label, and
-- a method wherein the method of detecting/discriminating as electrical, magnetic and optical changes on the surface of a biochip has a step of detecting/discriminating at least one of changes in current values, voltage values or resistance values on a biochip or electrode or changes in capacitance on the surface of a biochip, a step of magnetically detecting/discriminating a signal from a complex which forms a double chain or from label modifying a one or both of the probe nucleic acid or biochemical specimen of that complex, or from a complex and the aforementioned label, and a method of optically detecting/discriminating a signal from a complex which forms a double chain or from a label modifying one or both of the probe nucleic acid or biochemical specimen of that complex or from the complex and the aforementioned label.

Moreover, the present invention is a biochip constituted by a substrate or substrate analog having at least one electrode formed on its surface and having probe nucleic acids arrayed with one end fixed on the surface of the aforementioned electrode with each probe nucleic acid having a loop structure.

Moreover, a biochip is also proposed wherein in the biochip of the aforementioned configuration the arrayed probe nucleic acids
1) have a loop structure wherein the principal part which binds complementarily with the biochemical specimen is located on the substrate side,
2) have a loop structure wherein the free end not fixed to the surface of the electrode or the site capable of being modified with a label is located on the substrate side,
3) have a loop structure wherein a site modified with a first label which allows further modification with a second label is located on the substrate side, and
4) have a loop structure wherein a previously attached label is located on the substrate side.

Moreover, in the biochip of the aforementioned configuration a configuration is also proposed wherein the label is selected from metal fine particles (including Si), magnetic particles, ceramic fine particles, fluorescent labels, fluorescent dyes, dyes, chemical colorants and semiconductors, as well as a configuration wherein the substrate or substrate analog material is glass of semiconductor silicon and a configuration which allows embedding of the substrate or analog thereof into a separately prepared electrical circuit board.

### BRIEF DESCRIPTION OF THE DRAWINGS

Figures 1A and 1B are explanatory drawings showing the loop structure of probe DNA according to the present invention.
Figures 2A and 2B are explanatory drawings showing the loop structure of probe RNA according to the present invention.
Figure 3 is an explanatory drawing showing another loop structure of probe RNA according to the present invention.
Figure 4 is an explanatory drawing showing another loop structure of probe RNA according to the present invention.
Figure 5 is an explanatory drawing showing another loop structure of probe RNA according to the present invention.
Figure 6A is a slanted explanatory drawing showing the configuration of a biochip transport arm according to the present invention, while Figure 6b is a slanted explanatory drawing showing the configurations of a transport arm and a scanning device.
Figure 7 is a slanted explanatory drawing showing the configuration of another biochip according to the present invention.

### BEST MODE FOR CARRYING OUT THE INVENTION

In the present invention, a biochemical specimen is DNA, RNA and a nucleic acid-like structure, and a nucleic acid-like structure is literally a structure similar to a nucleic acid, such as a molecule in which a ribose or phosphodiester site of a nucleic acid has been modified or a peptide nucleic acid (PNA) in which a ribose-phosphoric acid framework has been replaced by an amide bond.

In the present invention, a probe nucleic acid is DNA, RNA and a nucleic acid-like structure, wherein for example a nucleic acid-like structure is similar to the above.

In the present invention, the configuration or formation method adopted to impart a loop structure to the probe DNA or RNA may be either one in which the loop structure is formed during the manufacturing process or after manufacture and then arrayed on the substrate, or one in which the loop structure is formed during arraying on the substrate, or in which the loop structure is formed after arraying on the substrate.

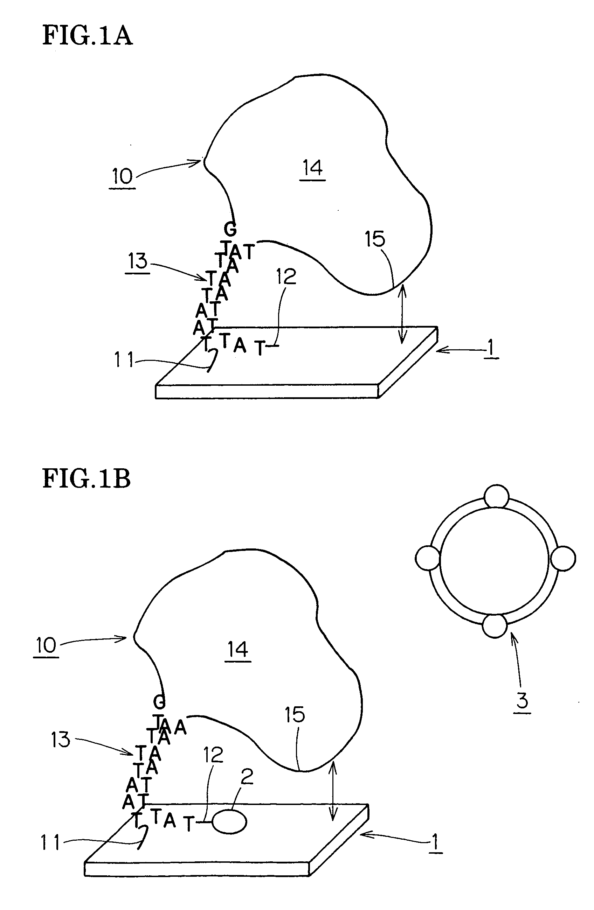

For example, a configuration can be adopted in which sequences of paired nucleotides capable of forming a complementary double chain are formed at two specific sites on the probe DNA, such as for example the A and T sites in the figure, and as shown in Figure 1A, bound section 13 is formed by a nucleotide sequence near fixed end 11 and a nucleotide sequence near free end 12 when probe DNA 10 is arrayed on substrate 1, forming loop 14 so that the free end 12 of probe DNA 10 is located on substrate 1 or near the upper surface of substrate 1.

Moreover, the example of Figure 1B is configured so that bound section 13 is formed by a nucleotide sequence near fixed end 11 and a nucleotide sequence near free end of the probe DNA 10 to form a loop 14 and probe DNA 10 arrayed on substrate 13 thereby forms a loop as in Figure 1A, whereby a site capable of being modified by label 3 (biotin base 2 at the free end in the figure) is located on substrate 1 or near the upper surface of substrate 1.

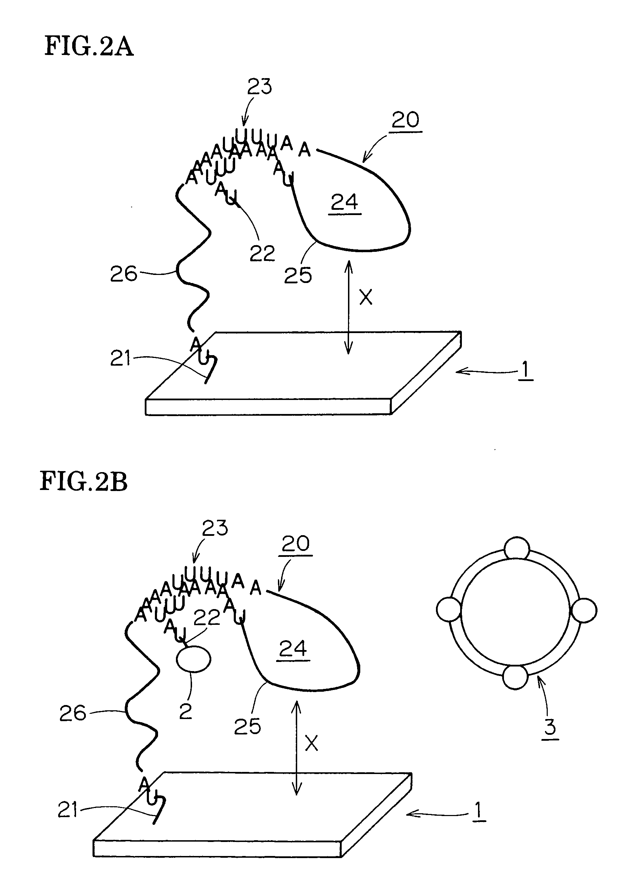

A variety of loop structures with bound section 13 located at the tip, the middle or the fixed end can be obtained by appropriately selecting the locations of the aforementioned sequences of nucleotides which pair to form the loop structure of the probe nucleic acid. For example, in the example of Figure 2 a nucleotide sequence which pairs with nucleotides at the free end 22 of probe RNA 20 is placed in the center of probe RNA 20.

In the example of Figure 2A the sequence of pairing nucleotides is placed in the middle of probe RNA 20 so that principal part 25 which binds complementarily with the biochemical specimen forms loop 24 which is directed more towards substrate 1 than in the example of probe DNA 10 in Figure 1.

In the example of Figure 2B, biotin base 2 on free end 22, which is modifiable with a label, is placed above substrate 1 in the same configuration as in Figure 2A, and as in the example of Figure 1B the biotin base 2 part which is modifiable with label 3, although distant from substrate 1, is surrounded by the nucleotide sequence which pairs with free end 22 of probe RNA 20 (that is, by central binding part 23), by loop 24 and by leg 26 between fixed end 21 and central binding part 23, so that if the dimensions of gap x between substrate 1 and loop 24 are the same as or smaller than those of label 3 modification by label 3 will be difficult regardless of how free end 22 and biotin base 2 are oriented with respect to substrate 1.

In the present invention a probe nucleic acid can be provided as described above with binding parts which form a loop so that that the principal part which binds complementarily with the biochemical specimen is arrayed within the loop, such as for example molecules or other binding parts which are sequences of paired nucleotides at any two sites in the probe nucleic acid, and it is obvious from the aforementioned explanation that any configuration can be adopted for the positions and loop structure of the binding parts which forms the aforementioned loop as well as for the orientation of the free end of the probe and the like.

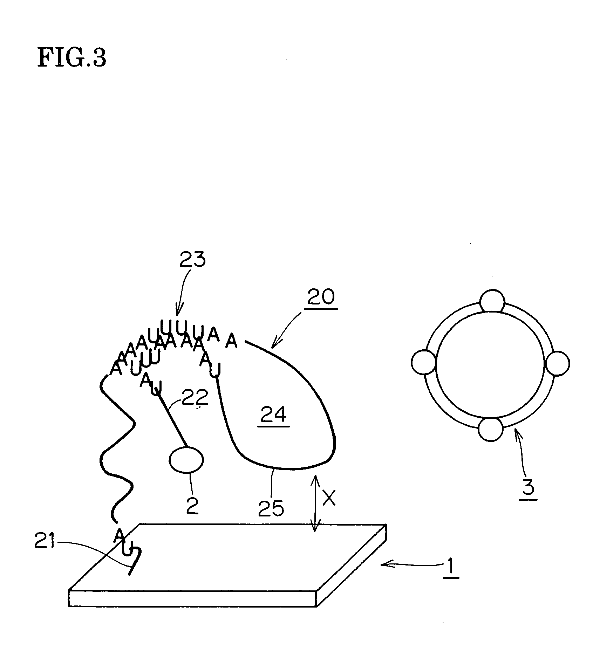

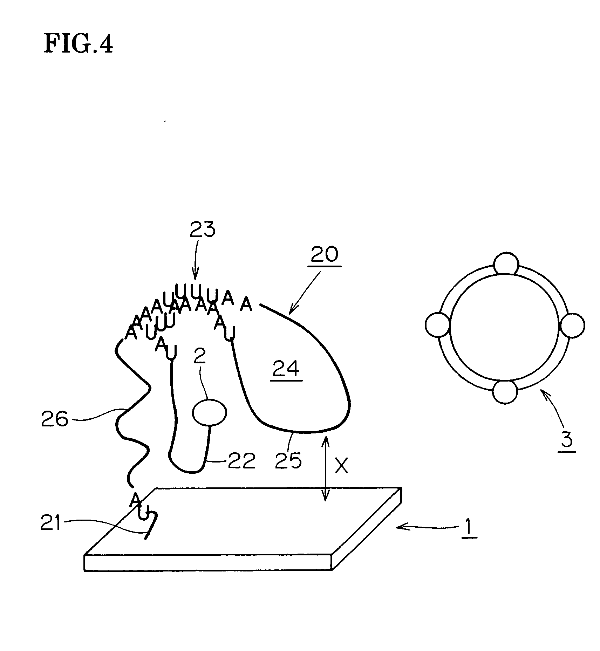

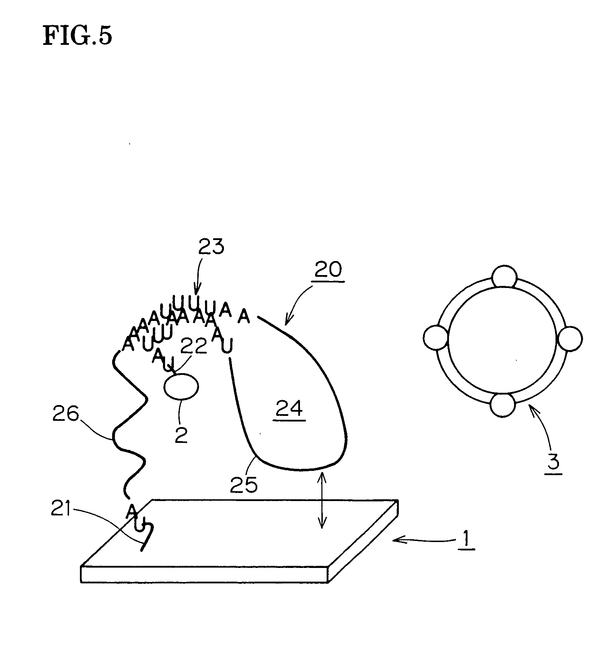

For example, a configuration in free end 22 extends further from binding part 23 so that free end 22 with biotin base 2 is closer to substrate 1 as shown in Figure 3, or a configuration in which free end 22 extends even further from binding part 23 in the same configuration as in Figure 3 and biotin base 2 is oriented towards binding part 23 as shown in Figure 4, or as shown in Figure 5 a configuration in which loop 24 is elongated to bring it closer to substrate 1 in the same configuration as in Figure 2B could be adopted.

As mentioned above, in contrast to that of Figure 1 the configuration of Figure 2 is provided with leg 26 so as to position binding part 23 in the middle of probe RNA 20, but since this leg is not particularly related to the pair-forming nucleotide sequence of binding part 23 or the principal part of loop 24 which binds complementarily with the biochemical specimen it need not necessarily be a sequence of nucleotides or the like but instead a variety of configurations can be adopted including a known nucleic acid-like structure or another molecule for purposes of setting the aforementioned gap x between substrate 1 and the loop structure. In addition, although loop 24 is formed by forming binding part 23 at the end of leg 26, for example leg 26 could be configured as a Y shape with binding part 23 and loop 24 on each, forming probe RNA 20 with one fixed end 21 and two loops stemming from leg 26.

In the present invention it is also possible to adopt a configuration having a loop structure in which a site modified by a first label capable of being modified by multiple second labels is located on the substrate or near it on the substrate side, and the location for the aforementioned binding part can be set appropriately so that the gap between the substrate and the loop is the same as or smaller than the outside diameter of the second label, to the extent that the second label cannot pass through and easily approach the first label.

Any nucleotide sequence configuration, whether short chain or long chain, can be adopted for the probe nucleic acid as long as the necessary hybridization is possible with the target biochemical specimen and as long as loop structures of various configurations can be formed as described above. Moreover, in the present invention the number of nucleotides is not particularly limited but a relatively long-chain configuration is desirable, and for example a sequence of 50 or 60 or more nucleotides which is fundamentally difficult to modify with a label is desirable from the standpoint of improving detection accuracy and decreasing noise during detection, but the present invention is still applicable if the number of nucleotides exceeds 100 or is even about 1000.

In the example given, sequences of pair-forming nucleotides are provided at two sites in the probe nucleic acid as the binding parts which form the loop in the present invention, but any molecules or structures other than nucleotide sequences can be used which can be placed or arrayed within the probe nucleic acid and which can form a loop by pairing, including those capable of intermolecular binding, for example, or means used for label modification.

The binding force in the binding parts can also be selected appropriately according to the nucleotides and molecules used and their positions and lengths, and factors such as temperature conditions during hybridization and label modification and binding force with other molecular structures (the specimen and the like) can also be considered in selecting the binding force of the binding parts.

Thus, the probe nucleic acid of the present invention is arrayed on the necessary electrodes and is configured with a loop structure as described above so that it can form a double chain only with the target biochemical specimen in the necessary hybridization operation. Moreover, because this loop is eliminated when a double chain is formed, only a probe which forms a double chain can be modified with a label.

Depending on the detection or discrimination method or the like including the label used in the present invention a variety of forms can be adopted for the substrate or analog thereof for forming the biochip as long as the probes can be arrayed and hybridization performed, including not only a substrate which is a pure plate but also a substrate configuration in which grooves, indentations and the like are formed by etching to provide necessary indentations and bumps, a configuration in which pins or other projecting parts are formed on the substrate, or a form or configuration using rods, three dimensional structures or other surfaces or the like, and electrodes can be formed at specific sites on these surfaces.

In addition to a glass substrate, resin substrate, silicon substrate or other solid material, a material such as a membrane (nitrocellulose or the like for example) can be used as the substrate in the present invention, and a substrate of any material or configuration, including a layered substrate, can be adopted as long as the probe nucleic acid can be arrayed thereon. When a variety of electrodes or other thin films are to be formed on the substrate it is desirable that the surface roughness be as smooth as possible.

Glass substrates which are easy to obtain and handle such as known borosilicate glass and the like can be used, and while they are easier to handle the thicker they are any thickness can be adopted according to the intended use of the biochip.

When forming an electrode film, known substrate washing and drying methods can be selected and combined for washing and drying the substrate or analog thereof, such as those used in manufacturing semiconductor wafers and various devices, including washing with various solvents, ultrasound washing in pure water, washing with various acid solutions, pure water washing, blow drying, spin drying and the like.

The electrode material can be formed on the substrate or other necessary surface, and any known electrode material can be adopted as long as the probe nucleic acid can be arrayed on the electrode film. It is also desirable to provide a gold, platinum, silver or other precious metal electrode film which is an electrode and also allows the probe nucleic acid to be arrayed easily.

There are no particular limits on the method of forming the film, but it is desirable to adopt methods used in etching, membrane formation and other semiconductor device manufacturing processes and which are also used in manufacturing the aforementioned substrate of the necessary form or the like. For example, in order to form an electrode and its lead in the necessary pattern and control the film thickness at a fixed thickness, a known method employing vapor phase epitaxy such as sputtering, ion plating, CVD or the like is desirable.

An under-layer can also be formed as appropriate in order to improve adhesiveness with the electrode film according to the material of the substrate used and the like. For example, a means can be adopted such as adding a Cr layer to a glass substrate or quartz substrate or surface modifying it with a silane compound.

In the present invention there are no particular limits on the step of arraying the probe nucleic acid on the substrate or other necessary electrode surface, and any known method can be adopted, including for example first washing the top of the electrode membrane with acid or pure water, and arraying in a saturated steam atmosphere using a buffer solution of the probe nucleic acid.

A solution adjusted to the necessary pH by mixing for example KH₂PO₄ and K₂HPO₄ can be used as the buffer solution. Alternatively, a solution or the like can be used which is compounded from a selection of known chemicals to achieve the desired pH, such as for example PBS, NaCl and Tris-HCl or NaCl, Tris-HCl and EDTA.

The method for detecting a biochemical reactant of the present invention is explained in detail below. To explain the basic step, they are:
-- a step of arraying a probe nucleic acid on a substrate or other surface,
-- a step of hybridizing a biochemical specimen with a probe nucleic acid which forms a loop structure,
-- a step of modifying with a label, either during or after hybridization, either or both of the biochemical specimen and the probe nucleic acid which have formed a double chain, and
-- a step of detecting/discriminating the aforementioned label.

In the step of arraying the probe nucleic acid on the substrate surface, the configuration of the substrate, configuration of the probe nucleic acid and arraying method are as explained above. Any configuration or method can be adopted for providing the probe with the loop, including either forming a loop structure during or after the manufacturing step and then arraying the probe on the substrate, or forming the loop structure while arraying the probe on the substrate, or forming the loop structure after arraying the probe on the substrate.

There are no particular limits on the step of hybridizing the biochemical specimen with the probe nucleic acid which forms a loop structure, and any known method can be adopted, such as for example first washing the substrate and then hybridizing using the biochemical specimen and a buffer solution. A solution in which the necessary pH is maintained by a mixture of NaCl, Tris-HCl and EDTA for example can be used as the aforementioned buffer solution.

The step of modifying with a label, either during or after hybridization, either or both of the probe nucleic acid and biochemical specimen which have formed a double chain is a step of literally attaching a necessary label to the probe nucleic acid or the biochemical specimen or to each of them once they have formed a double chain, and there is no particular need for the label to be an intercalator capable of selectively penetrating the bound part between the two as a complex forming a double chain, but an intercalator can also be adopted or used together with the aforementioned label.

In the present invention the aforementioned label can be any which can modify a probe nucleic acid or biochemical specimen and any which can be re-modified with any of the following labels, such as metal particles including Si, magnetic particles, ceramic particles, a fluorescent label, a fluorescent dye, a dye, a chemical colorant or a semiconductor or else a chromosome, sugar chain, protein, nucleic acid, enzyme, cells or the like which is used for detecting biochemical specimens.

In terms of electrical characteristics the label need not be conductive, and in the biochip of the present invention any configuration can be adopted for the label such as using a label which can be detected according to electrical changes in current, voltage, resistance, capacitance and the like caused by the presence or absence of the label, or a label capable of causing magnetic changes in addition to the electrical changes due to the presence or absence of the label, or a label capable of causing both electrical and optical changes due to the presence or absence of the label.

Metal particle labeling uses fine particles of various metals such as Au, Al, Ti, Cr, Mn, Fe, Co, Ni, Cu, Zn, Mo and the like including Si, and in particular any form, dimensions and uniformity of the fine metal particles can be selected as appropriate. Although this is not a necessary condition, fine particles of 5 µm or less or preferably 1 µm or less which have a specific uniform grain size in the range of a few to a few hundred nm and which can be stably obtained industrially are desirable from the standpoint of allowing the necessary part of the probe or specimen to be modified and allowing formation of films on the surface of the fine particles and the like.

In addition to iron oxide fine particles which are common in such uses as magnetic powders, iron oxide pigments, magnetic fluids and the like, carbon fine particles which are used as magnetic toners and magnetic inks can be used for magnetic particle labeling, as can a number of magnetic materials which are included among the aforementioned metal particles and the ceramic particles described below. Moreover, iron oxides and other oxides and alloys and various other metal compositions and composite particles have been proposed as magnetic powders for magnetic recording media, and these are desirable because they have a uniform shape and specific particle size in the range of a few to a few hundred nm and can be stably obtained industrially. In addition, the colored magnetic powders used as magnetic inks and toners exhibit a variety of magnetic properties and hues depending on the combination of material, size and shape of the core magnetic powder and the material, thickness and number of layers of surface film, so that magnetic detection and optical detection can be performed simultaneously.

Ceramic particle labeling can be accomplished with any known form of particle including spheres and intrinsic crystals, but from the standpoint of allowing film formation on the surface of the fine particles 5 µm or less or preferably 1 µm or less is desirable, and depending on the detection method SiO₂, TiO₂, ZrO₂, Al₂O₃, MgO or other ceramic fine particles in the range of a few nm to a few hundred nm with a uniform specific particle size which can be stably obtained industrially are desirable.

Any known configuration can be adopted for fluorescent labeling, and as described below modification can be accomplished with a chromosome, sugar chain, protein, nucleic acid, enzyme, cells or fine particles or the like tagged with a commercially available fluorescent dye using the properties of the label itself or an antigen-antibody reaction as described below. Because fluorescence itself is not required in the present invention, the chromosomes, sugar chains, proteins, nucleic acids, enzymes and cells used in fluorescent labeling or the like can also be used as is. A non-fluorescent dye can also be used as a label.

In the present invention a chemical colorant is a substance used in methods of chemical coloring such as enzyme methods in which a substance involved in a coloring reaction is attached as a label, and this can be easily accomplished for example by preparing sites modified with a biotin derivative.

In the present invention any known methods can be adopted as the method for modifying the probe nucleic acid with the various labels described above, such as for example methods using the properties of the metal fine particles or other particles themselves or methods using an antigen-antibody reaction such as known methods of fluorescent label modification. Modification can be facilitated by employing a solution of uniformly dispersed fine particles, such as a neutral colloidal solution.

Labeling can also be accomplished by modifying the tip of the probe nucleic acid with biotin and then exploiting the strong biotin-avidin binding force using metal or ceramic fine particles or the like coated with streptoavidin. Modification can also be achieved by adding IgG or anti-protein to the tip of the probe nucleic acid and exploiting the antigen-antibody reaction using the aforementioned fine particles or the like coated with protein.

Moreover, the biochemical specimen can also be modified with the various aforementioned labels, using any of the known methods described above for modification as long as the necessary site of the aforementioned specimen can be appropriately labeled, and any method including those described above can be used to modify the tip in particular.

In the present invention the method of detecting/discriminating a complex of the probe nucleic acid and biochemical specimen which have formed a double chain after hybridization as electrical changes on the surface of the biochip substrate or the like is for example a method of detecting at least one of changes to current applied to the substrate or to voltage or resistance values or changes to capacitance on the substrate or other surface, and distinguishing the aforementioned complex according to the aforementioned changes before and after hybridization.

Specifically, in a configuration in which the biochip is divided into a plurality of detection spots (stages) with each spot having a specific number of electrodes, and a number of probe nucleic acids which form loops are arrayed on each electrode with a different probe for each spot, a specific current is applied (voltage is applied) to the substrate or the like or to each electrode as alternating current, direct current or pulse current before hybridization either under dry conditions or under wet conditions such as immersion in a specific buffer, and the current value I, voltage value V, resistance value R and capacitance value C are measured between specific electrodes.

Next, hybridization is performed with the specified biochemical specimen, specific current is again applied (voltage is applied) either in a specified buffer immediately afterward or after washing or else after washing and drying, the same measurements are performed as above, and production of complexes of the probe nucleic acid and biochemical specimen forming a double chain by hybridization on each probe electrode can be detecting by observing and analyzing changes in the measurements.

In order to detect whether or not complexes of the probe nucleic acid and biochemical specimen are produced on each electrode, it is also possible to adopt a configuration such as one in which an electrical measurement circuit or electrical element circuit is formed on the biochip which measures electrical changes in the substrate or other surface of the biochip as current I, voltage V, resistance R, capacitance C or some combination of these, and which is connected to the aforementioned probe electrode.

Furthermore, in order to detect whether or not complexes of the probe nucleic acid and biochemical specimen are produced on the probe electrodes, another probe electrode or sensor capable of detecting the current I, voltage V, resistance R, capacitance C or the like to be supplemented can be placed near the surface of the biochip and the surface scanned to assess changes in at least one of the value of current I applied between specific electrodes or the voltage V, resistance R or capacitance C before and after hybridization and detect the presence or absence of complex production accompanying hybridization.

In the present invention the method of detecting/discriminating a complex of the probe nucleic acid and biochemical specimen forming a double chain after hybridization which has been modified with a label added to either or both of the probe nucleic acid and biochemical specimen either in advance or after hybridization as electrical, magnetic, optical, electromagnetic, electro-optical or electro-magneto-optical changes to the substrate or other surface of the biochip of course includes detection of electrical changes accompanying complex formation as described above, but this method is also capable of assessing the presence or absence of such a complex as changes to at least one of current value I, voltage value V, resistance value R or capacitance value C which occur between specific electrodes due mainly to the physical properties of the selectively used label.

Detection of electromagnetic changes here signifies assessing electrical changes due to either or both of the aforementioned probe nucleic acid or biochemical specimen or to an additional label added thereto and also detecting by a magnetic detection means the magnetism or the like of a label added to the complex itself or to either or both of the probe nucleic acid or biochemical specimen thereof. Consequently, it signifies of course detecting the magnetism or the like of the label by a magnetic detection means but also applying voltage between specific electrodes or applying a magnetic field to the biochip and detecting by a magnetic detection means changes in acquired electrical characteristics or magnetic characteristics or both of the complex or label or both.

For example, this signifies of course detection when magnetic fine particles for magnetic recording media are used as the label and a magnetic head is used as the detection probe to detect magnetic changes after application of a specific magnetic field, but also detection as changes in current value I, voltage value V, resistance value R or capacitance value C between specific electrodes after application of voltage, a magnetic field or both when metal or ceramic particles are used as the label, and electrical changes and magnetic changes can be detected either separately or simultaneously.

Detection of electro-optical changes here signifies assessing electrical changes due to either or both of the aforementioned probe nucleic acid or biochemical specimen or to an additional label added thereto and also detecting by an optical detection means signals from a label added to the complex itself or to either or both of the probe nucleic acid or biochemical specimen thereof. Consequently, it signifies of course detecting the optical characteristics of the label by an optical detection means but also applying voltage between specific electrodes or applying a magnetic field to the biochip and detecting in a similar way by an optical detection means changes in acquired electrical characteristics or optical characteristics or both of the complex or label or both.

For example, this signifies of course detection when metal particles or ceramic fine particles are used as the label and a CCD is used as the detection probe to detect changes in the signal emitted by the label after application of a specific voltage or magnetic field, but also detection as changes in current value I, voltage value V, resistance value R or capacitance value C between specific electrodes after application of voltage or a magnetic field or after exposure to laser light or after all these operations, and electrical changes and optical changes can be detected either separately or simultaneously.

Moreover, detection as electro-magneto-optical changes which are a combination of the aforementioned electromagnetic changes and electro-optical changes is obviously also possible. For example, magnetic fine particles have been developed which are magnetic but also emit light at a specific wavelength, and using these and a suitable combination of the aforementioned steps and the like, signals from the label of a complex and the probe nucleic acid or biochemical specimen of a complex or both can be easily assessed as electrical, magnetic and optical changes.

Means of optically detecting/discriminating the various labels near the surface of the substrate can be used at the same time as the aforementioned electrical detection on the substrate of the biochip. For example, light scattering, SPR spectrometry, chemical coloring, fluorescent detection, microscopy, image processing or visual observation can be adopted.

In the case of labeling with metal particles or ceramic fine particles, the light scattering method allows detection of scattered light from laser light which is reflected from fine particles at a specific wavelength. To give an example of a common configuration, for example a detection chip substrate of the present invention having a thin gold film is mounted on a prism, the conditions are set so that He-Ne laser light which is directed at the reverse side of the substrate from one side of the prism is totally reflected to the other side, and scattered light can then be detected by a CCD camera which observes the detection chip substrate from above.

It is also possible to appropriately select a known detection method according to the type, size, properties and other conditions of the ceramic fine particles, such as by detecting the specific color of ceramic fine particles themselves in visible light or by illuminating ceramic fine particles of a specific particle size with light at a specific wavelength so that they emit light of a specific color which is then detected.

In SPR spectrometry changes in refractive index are detected on the surface of a thin film of precious metal, and for example if a gold electrode is formed on a glass substrate, a probe nucleic acid is fixed on the gold thin film electrode and the probe nucleic acid then forms a double chain by hybridization with a target RNA or other nucleic acid in a sample, the SPR angle (the incidence angle at which reflectance is lowest) will shift due to changes in the refractive index on the gold thin film electrode, and since the shift in SPR angle corresponds to the amount of hybridized target specimen a target specimen can be quantitatively analyzed by SPR measurement.

Moreover, in SPR spectrometry it is also possible to amplify the aforementioned signal by label modification. To explain in more detail, if a probe nucleic acid is arrayed on the precious metal thin film surface of a substrate to create a biochip and the probe is modified during or after hybridization with a label of precious metal colloid, metal particles or the like, as described above the label will be unable to attach to a probe which has been modified with an attachment site for a label at the tip if the probe assumes a loop (hairpin) structure, and detection accuracy will be fundamentally improved because probe which assumes this loop structure will hybridize selectively with the target specimen because of its three-dimensional structure.

Moreover, the loop of the probe nucleic acid is eliminated by the hybridization reaction with the target specimen so that the hybridized probe nucleic acids are selectively modified with the label, allowing highly accurate detection because the shift in the aforementioned SPR angle can be amplified. In addition, the step of detecting by SPR spectrometry a target specimen which has formed a double chain after hybridization can be performed in either liquid or atmosphere. Any known configuration can be adopted for the measurement system in the SPR method.

Tissue immunoblotting can be adopted as the chemical coloring method. This is accomplished by mixing in advance suitable proportions of avidin having four biotin binding sites and peroxidase (HRP) which has been biotinylated at a plurality of sites to form a complex (ABC) comprising multiple HRPs and partly retaining the biotin binding sites of avidin, and detecting labeled antigen by reacting this complex (ABC) with biotinylated antibodies already bound to the target antigen in tissue. This can be easily applied for example by preparing sites modified with biotin in the probe. Known tissue immunoblotting methods such as APR can also be applied

In fluorescent detection methods a variety of fluorescent dyes can themselves be attached to specific sites on the probe nucleic acid or label, or a chromosome, sugar chain, protein, nucleic acid, enzyme, cells, fine particles or the like tagged with fluorescent dye can be used to modify the probe nucleic acid or biochemical specimen using the properties of the label itself or an antigen-antibody reaction as described above and detected. Fluorescent imaging detection systems using a combination of microscopes and CCD cameras, confocal microscope systems and the like have been proposed as well as detection systems which employ a combination of various optical devices and imaging devices, and known detection methods and devices can be selected appropriately according to the type of aforementioned fluorescent label, the properties and the like of the fluorescence itself and the conditions. A non-fluorescent dye can also be detected in the same way.

For microscopic observation known fluorescence or scattered light detection systems have been proposed including imaging detection systems using a combination of microscopes and CCD cameras, confocal microscope systems, metallographic microscopes and the like or else a variety of optical devices and imaging devices used in combination, and these can be used as is.

Image processing includes known image processing methods such as enlarging, sharpening or coloring an image scanned from a camera or microscope so that it can be checked visually on a display, or contrast or other image processing to allow specific shapes or particle dimensions to be detected by the software.

Visual observation is possible because on the biochip of the present invention only a probe nucleic acid and target specimen which have formed a double chain by hybridization can be modified with a particle label. The presence or absence of the target specimen is detected by visually observing aggregations of the various label particles described above.

In the present invention the label has a particular color or form as described above which can be observed in aggregate, and when in detection the probe or specimen is labeled with a first label and second label these are observed. Moreover, when the first label is used as a target for modification with the second label, both the first label and second label are observed, or if the first label cannot be observed only the second label is observed.

An observable aggregate of label particles can also be produced by 2-stage modification involving modification with a first label as described above followed by modification with a second label targeting the first label, or multi-stage labeling by repeated modification of a previously attached label.

In the present invention it is possible to quantitatively assess the condition of the probe on each electrode before the hybridization operation or the like by first label modifying the probe nucleic acid arrayed forming a loop structure on the biochip, and there are no particular limits on the label modifying this probe which may be any of the aforementioned. Any part of the loop structure of the probe nucleic acid may be modified such as the loop or leg shown in the figure or the like, and modification may be added at any step in the process of making the biochip such as when the probe nucleic acid is arrayed on the electrode or afterwards, as long as the condition of the probe arrayed on each electrode of the biochip before hybridization can be quantitatively assessed.

For purposes of improving detection accuracy or quantification in the present invention, the label attached in advance to the probe nucleic acid on the biochip and the label used to modify the biochemical specimen after hybridization may also be of different types, such as a label which can be detected magnetically and a label which can be detected optically. In this way, the amount of probe nucleic acid can be assessed using a magnetic sensor at the initial detection stage of the biochip while the biochemical reactant can be detected using a scattered light sensor after hybridization. Moreover, using ceramic particle labels as the aforementioned two labels the analytic function can be improved for example by changing the outer particle diameter so that multiple kinds of light are used to observed the same strength of scattered light, or a target label such as the label modifying the probe nucleic acid or the label modifying the biochemical specimen can be detected separately.

Moreover, when the biochip of the present invention has been labeled in advance the relative quantities of probe nucleic acids fixed to each electrode arrayed on the chip as described above can be accurately assessed beforehand, so that the correlations, analyses and other measurement results based on measurement signals from the biochemical specimen on each electrode which are obtained by various aforementioned electrical, magnetic and optical means after each operation can be corrected by the known relative quantities of probe nucleic acid present on each electrode, allowing the biochemical reactant to be quantitatively detected with high accuracy.

In addition, by appropriately selecting the configurations, dimensions or types of labels so that the label modifying the probe can be clearly distinguished from the label attached after hybridization or the like, it is possible without performing specific measurements before hybridization as described above to quantitatively assess the probe on each electrode after completion of hybridization or subsequent label modification by measuring one or more of electrical, magnetic and optical changes for example. Consequently, if for example the loop, leg or other site of the probe nucleic acid is modified with a fluorescent dye or minute magnetic particles and the biochemical reactant forming a double chain is modified with ceramic particles with a relatively large particle size, the relative amounts of probe on each electrode and the relative amounts of biochemical reactant forming a double chain can be detected or discriminated in only one measurement operation following completion of all operations, improving the operational convenience.

In the example explained above the probe nucleic acid was modified in advance with a label in order to assess the relative amounts of probe nucleic acid on each electrode in the biochip of the present invention, but of course the relative amounts of probe nucleic acid can be assessed using a method in which the aforementioned electrical changes are measured without label modification. When the probe nucleic acid is not label modified in advance, the relative amounts before hybridization can be calculated based on electrical changes, or else electrical, magnetic and optical changes can be measured together after hybridization, modification and other operations and used to derive the relative amounts of probe nucleic acids.

In the present invention various configurations can be adopted for the form of the biochip as described above, and one example of a configuration of explained here. A rectangular silicon substrate and glass substrate are used here for the biochip, which is handled inside a carrying case which is not illustrated, and a configuration was adopted in which the biochip is taken in and out of a normal lidded container as the case and a configuration such as a commercial DVD-RAM or other carrying case in which, when the carrying case is inserted into a scanning device for purposes of detection, only the biochip inside the external case is exposed to the scanning device. That is, it was assumed that handling would be either by hand or automatically or semi-automatically by machine.

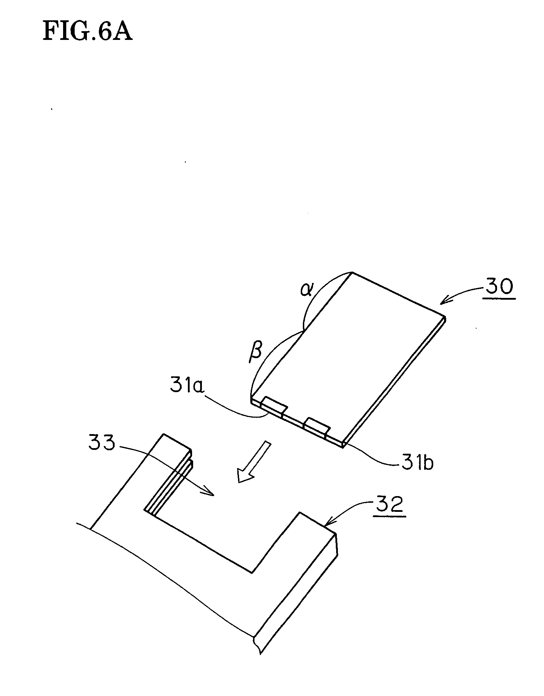

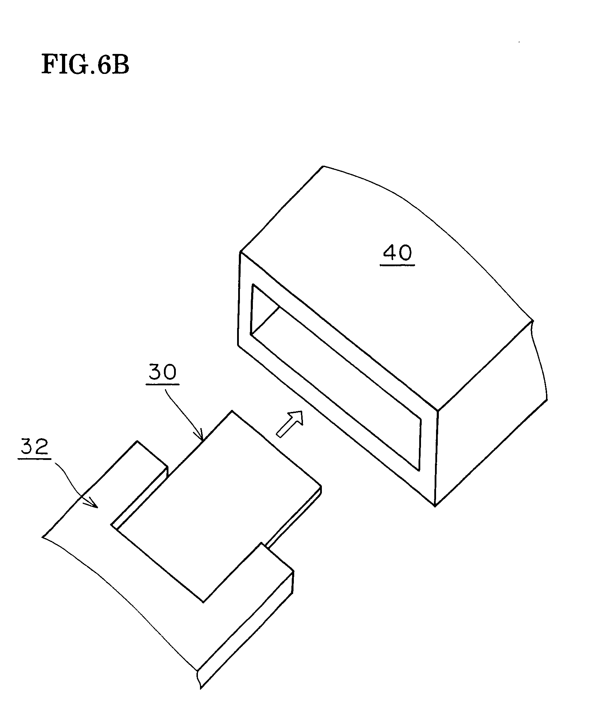

Biochip 30 in Figure 6A is removed from its carrying case and placed on transport arm 32 for purposes of semi-automation, and a configuration is adopted here in which lead terminals 31a and 31b of biochip 30 are inserted into the connection terminals inside U-shaped holding part 33 of transport arm 32 and held so as to allow passage of current. With biochip 30 held in the groove of U-shaped holding part 33 of the transport arm 32 shown in Figure 6B, a specific voltage or current is applied through arm 32 in this dry state, and electrical measurements of current and resistance values and the like can be performed with the necessary probe arrayed on each electrode of the chip.

Lead terminals 31a and 31b are not shown in detail, but a lead film is formed in a specific pattern on the substrate surface, with lead terminal 31a leading from a plurality of electrodes with arrayed probes in region a, which is the half of biochip 30 opposite the terminal, and lead 31b leading from a plurality of electrodes with arrayed probes in region b, which is the other half of biochip 30. The surface of biochip 30 is divided into region a and region b here in order that the necessary measurements before and after hybridization can be accurately correlated.

That is, the electrodes and various necessary probe nucleic acids are arrayed appropriately in exactly the same pattern in the two regions, and only that part of region a is dipped in buffer solution to hybridize the specific biochemical specimen, or modification with a label may also be performed, but region b is not subjected to either label modification or label modification. After completion of the aforementioned hybridization region a is washed and dried, and the necessary voltage or current is applied to both regions in a dry condition through arm 32, so that electrical measurements of current, resistance value and the like can be performed for both un-hybridized region b and hybridized region a.

Moreover, the biochip is inserted into scanning device 40 while being held by transport arm 32 as shown in Figure 6B. The device contains either an electrode probe, CCD probe, magnetic head or other electrical, optical or magnetic probe, or a combination of these which can be brought from above close to the electrodes having probe nucleic acids on the surface of biochip 30, so that in addition to the aforementioned measurements by current from transport arm 32 electrical, optical and magnetic measurements can be performed before and after hybridization. Thus, by comparing these measurement results before and after hybridization it is possible to easily detect the presence or absence of a complex of a probe and biochemical specimen forming a double chain after hybridization, or in other words a biochemical reactant.

In the example described above one main surface of the substrate is divided into two, but it could also be further divided or both surfaces given the same configuration, or else the regions could be divided by slits or a plurality of strips or bars could be used without division into regions. A configuration could also be adopted in which the aforementioned region b was sealed with a lid or container part during hybridization.

When region a of the biochip was subjected to the aforementioned hybridization operation the other region b was not subjected to any another operation in the previous example, but this region could be immersed in a similar buffer containing no biochemical specimen for use as a negative control, and in this way the presence or absence of the specimen can be easily detected in the aforementioned operation. Moreover, two biochips 30 could be used and divided into a region without buffer operation, a negative control region, a known concentration region and the test sample region.

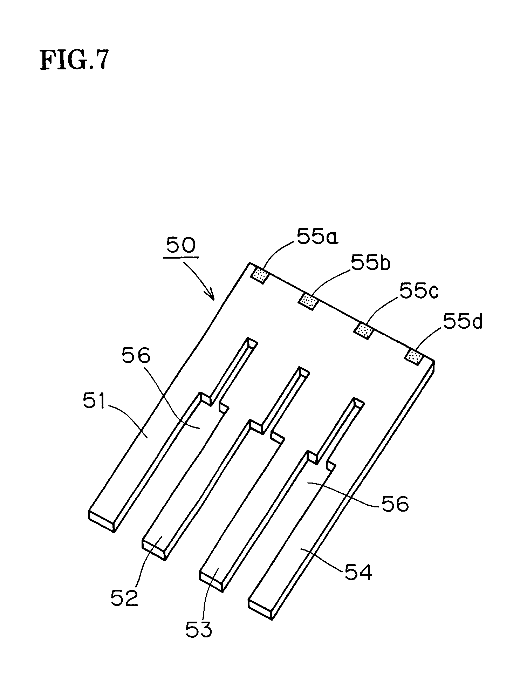

In addition, in the configuration shown in Figure 7 a plate-shaped biochip 50 is divided into four by three slits to create stick-shaped chip regions 51 through 54, the same electrode patterns are formed on each of chip regions 51 though 54, and lead terminals 55a through 55d are provided at the other end of chip 50 which connect to the electrodes of chip regions 51 through 54, respectively. The operation of arranging the necessary probe nucleic acids on the necessary electrodes is repeated for biochip 50 so that probe nucleic acids of the same kind are arranged under the same conditions on each of chip regions 51 through 54. Next, operations can be performed simultaneously under the same conditions using for example chip region 51 as the blank region which is not even dipped in buffer, chip region 52 as the negative control region which is dipped in buffer not containing the biochemical specimen, chip region 53 as the known concentration region which is dipped in the same kind of buffer containing a known concentration of the biochemical specimen and chip region 54 as the test sample region.

It is also possible to use a plurality of stick-shaped biochips formed individually with the same electrode pattern, and in addition to simultaneously arraying probe nucleic acids on each electrode of the chip surface and performing the same operations as in the example above, it is possible for example to perform several different hybridization operations with different concentrations of the biochemical specimen on a plurality of biochips, separate these into those with label modification and those without, and perform the aforementioned measurements with these plurality of biochips mounted on the transport arm to measure hybridization with greater quantitative accuracy.

### EXAMPLES

### Example 1

The object of detection was about 856 bp of DNA surrounding a mutation in the Campylobacter jejuni O-19 serum gyrB gene, and 60 central nucleotides of RNA which bound complementarily to this at the central part was prepared as probe RNA. Three types of probe DNA were prepared for purposes of comparison--30 terminal nucleotides of DNA which bound complementarily with the aforementioned DNA at the terminus, 30 central nucleotides of DNA which bound complementarily with the central part, and 60 central nucleotides of DNA. The 30-nucleotide probes were configured so as not to form loops within the same chain, and the 60-nucleotide probes so as to form loops within the same chain.

A glass substrate was used as the substrate for preparing the biochip. This was ultrasonically washed in acetone, methanol and ultra-pure water, surface washed for 20 seconds with 10% hydrofluoric acid, washed again ultrasonically in acetone, methanol and ultra-pure water and dried with nitrogen gas.

Next, after formation of a resist layer in a specific pattern, a Cr layer about 1 nm thick was formed on the glass substrate with a sputtering device (ULVAC), followed by formation of an Au layer about 50 nm thick. The resist layer was then removed to obtain an electric circuit with a configuration of Au film electrodes arrayed in the necessary pattern on a substrate and connected by Au film connections. In addition, an insulating mask layer was applied to parts of the circuit pattern, such as the Au film connections, other than the Au film electrodes on which the probe nucleic acids were to be arrayed.

The aforementioned substrate was immersed for about 1 hour in concentrated sulfuric acid, and washed in ultra-pure water. Next, a probe RNA,DNA-D-BFR solution (KH₂PO₄, K₂HPO₄, pH 7.0) in which the 3' end of the probe RNA and DNA was modified with an SH (thiol) group and the 5' end with a biotin group was dripped onto the substrate, which was left for about 15 hours in saturated steam to attach the probe RNA and DNA to the Au film electrodes of the glass substrate, creating the biochip of the present invention.

After washing with R-BFR solution (NaCl, Tris-HCl, pH 7.4) and H-BFR solution (NaCl, Tris-HCl, EDTA, pH 7.4), an H-BFR solution of specimen DNA with a specific concentration was dripped and left for about 16 to 24 hours to perform hybridization.

After hybridization had been performed using the aforementioned DNA chain with respect to 4 spots of the probe of the present invention and a comparative probe, the probe was modified with fluorescent fine particles labeled with avidin. Next, hybridization was detected or in other words fluorescence was detected with a fluorescent inverted microscope and a CCD camera.

Four probes of the same kinds were also modified in the same way without hybridization with a fluorescent label of fluorescent fine particles coated with avidin, and the fluorescence was then detected.

In the case of the 30-nucleotide probe DNA fluorescence was observed whether or not there was hybridization with the target DNA, confirming that because they lacked the loop structure they could normally be modified with fluorescent labels, making detection of the target DNA difficult.

In the case of the 60-nucleotide probe DNA and probe RNA, however, modification with the fluorescent label could not be achieved at all before hybridization with the target DNA, and strong fluorescence could only be observed from probe which had formed a double chain after hybridization, confirming that the 60-nucleotide probes have a loop structure and only the hybridized probes can be modified with a label at the tip, facilitating detection of target DNA.

It was also confirmed because DNA-RNA binding is more stable than DNA-DNA binding, detection sensitivity is greater in the case of the probe RNA of the present invention than in the case of the 60-nucleotide probe DNA used for comparison.

Moreover, after the aforementioned detection of the fluorescent label the biochip was washed and dried, and then mounted on a transport arm and set in a scanning device equipped with electrode probes as shown in Figure 6 to measure voltage, current and capacitance. These measurement results were observed in contrast with the measurement results of voltage, current and capacitance for the aforementioned dry run without hybridization or fluorescent labeling and with those with fluorescent labeling but without hybridization.

In a comparison with the comparative spots the electrical changes due to the presence or absence of hybridization and presence or absence of a fluorescent label matched the aforementioned measurement results for the fluorescent label itself, confirming that the biochip could detect hybridization as electrical changes. When the aforementioned operation was also performed using varying amounts of the target DNA during hybridization, changes in the signal correlating with the amount of target DNA were detected, confirming that quantitative detection is also possible.

Moreover, it was confirmed that more accurate quantitative detection of the hybridized probe could be achieved by equipping the aforementioned scanning device with an optical detector employing the aforementioned CCD camera and taking electrical measurements of the biochip mounted on the transport arm simultaneously with optical detection of the fluorescent label.

### Example 2

RNA was used in place of the DNA of Example 1 as the object of detection. That is, about 800 nucleotides of RNA prepared by in-vitro RNA synthesis from the gyrB gene (Campylobacter jejuni) was purified and used.

60 nucleotides of DNA which bound complementarily in the center with the aforementioned synthetic RNA was used as the probe DNA, and was arrayed under similar conditions and on a similar glass substrate as in Example 1 to prepare a biochip.

In the gold colloid modification method adopted here for attaching the label the aforementioned biochip was washed after hybridization with R-BFR solution and gently dried with nitrogen gas, and gold colloid (particle size 10 nm, SIGMA) coated with avidin was dripped and left for 1 to 3 hours in saturated steam to modify using the specific binding of biotin and avidin.

Moreover, metal fine particle modification was performed using a pH 7.4 colloidal solution of Fe fine particles with a mean particle size of about 1 µm coated in advance with avidin in order to achieve biotin-avidin binding with the 5' end of the probe.

Hybridization was performed as in Example 1 using the aforementioned RNA, followed by gold colloid or metal fine particle modification, followed by SPR measurement. For purposes of comparison, an H-BFR solution having no target RNA was reacted at the same time with the probe DNA, and subjected to SPR measurement following label modification. SPR measurements were performed immediately after the biochip had been washed in R-BFR solution and gently dried with nitrogen gas. SPR measurement was performed using an imaging system.

As a result, it was confirmed that gold colloid modification and metal fine particle modification were selectively possible only in the case of the probe DNA which had hybridized with the target RNA, allowing SPR angle shift amplification and therefore allowing detection of the biochemical reactant created by hybridization. In the same way, modification was also selectively possible only in the case of probe DNA which had hybridized with target RNA when modification was with streptoavidin by the same methods used for the aforementioned gold colloid modification.

For purposes of comparison, modification was attempted with an Fe fine particle label coated with avidin without performing the aforementioned hybridization. However, no modification with the Fe fine particle label was possible before (without) hybridization with the target RNA, and the SPR measurements after modification were obviously different from those obtained when modification with the Fe fine particle label was performed after hybridization.

Moreover, cases with and without the target RNA were established when performing the aforementioned hybridization, and modification with Fe fine particle colloid was performed, followed by observation by differential interference microscopy. When the target RNA was present many Fe fine particles were observed, while without the target RNA almost no Fe fine particles were observed. The presence of the target RNA could also be distinguished clearly with the naked eye in the case of any of the aforementioned biochips according to the grainy appearance of the surface.

Furthermore, after detection of the aforementioned metal particle label as in Example 1, the biochip was first washed and dried and then mounted on a transport arm as in Figure 6 and set in a scanning device equipped with an electrode probe to measure voltage, current and capacitance. These measurement results were observed in contrast with the measurement results of voltage, current and capacitance for the aforementioned dry run without hybridization or metal particle labeling and with those with metal particle labeling but without hybridization.

The differences and degree of differences in electrical changes due to the presence or absence of hybridization and presence or absence of metal particle labeling matched the SPR detection results from the metal label itself, so that in other words the amount of shift in SPR angle corresponded to the amount of the hybridized target specimen, confirming that the shift in SPR angle exhibits the same trend as the amount of change in electrical measurements, and confirming that the biochip is capable of quantitatively detective hybridization as electrical changes.

### Example 3

In place of the aforementioned gold or Fe fine particles used in Example 2, the probe DNA was modified with pH 7.4 colloidal silica, using silica particles which were coated in advance with avidin so that they would bind by biotin-avidin binding with the 5' end of the biotin-modified probe. A variety of particle sizes between 100 nm and 800 nm were present in the colloidal silica.

In the mechanism for detecting hybridization in the scanning device, a detection chip was mounted on a prism, He-Ne laser light was directed at the underside of the substrate from one side of the prism and totally reflected to the other side of the prism, and the strength of scattered light was detected by a CCD camera observing from the upper surface of the biochip.

For purposes of comparison, modification was performed without the aforementioned hybridization using a silica fine particle label coated with avidin. In the results, no modification with the silica fine particle label occurred before hybridization with the target RNA, and no scattered light was observed, but after hybridization strong scattered light was observed from the probe DNA which had hybridized with the target RNA. Moreover, the target RNA could be detected in the same way regardless of the size of the silica particles.

Next, a biochip was prepared as in Example 1 having the separation of surface regions shown in Figure 7, and chip region 51 was dipped in the same buffer as in Example 2 without specimen and then modified with the aforementioned silica particle label, chip region 52 was hybridized as in Example 2 using the same buffer having a known amount of the specimen, chip region 53 was hybridized as in Example 2 using the same buffer having a known amount of the specimen and then modified with a silica particle label, and chip region 54 was hybridized with the target RNA using a test buffer solution as in Example 2 and then modified with the aforementioned silica particle label. Each region was then first washed and dried and then mounted on a transport arm as shown in Figure 6 and set in a scanning device equipped with an electrode probe and CCD camera to detect the strength of scattered light and measure voltage, current and capacitance.

In addition to the aforementioned measurement results and measurement results for previously measured probe DNA arrayed as is, detection results were obtained from four sets of simultaneous measurements of voltage, current and capacitance for region 51, which was subjected to hybridization and silica particle labeling as a negative control, region 52, which was hybridized with a known amount of the target specimen, and regions 53 (known quantity) and 54 (sample), which were hybridized with the target specimen which was the object of testing and modified with a silica particle label, and for strength of scattered light from the four regions.

Biochips were also prepared with a configuration having the same electrode patterns as the regions of Figure 7 in two locations each on individual stick-shaped substrates, the operations described above were performed along with hybridization with the target RNA, followed by modification with a silica particle label, after which the biochips were washed and dried and set in the aforementioned scanning device to detect the strength of scattered light and measure voltage, current and capacitance. Since each chip had two regions one was subjected to the aforementioned operations and one was not, and 5 buffer solutions were prepared containing different pre-specified amounts of the target RNA for purposes of hybridization, and five hybridizations were performed with these buffer solution using 5 biochips.

In the measurement results a steady correlation was discovered between concentration of target RNA and changes in voltage, current and capacitance and changes in strength of scattered light in the presence of probe which had formed a double chain by hybridization with the target specimen and which had also been modified with a silica particle label, confirming that quantitative measurement was possible by comparison with the measurement results for the aforementioned hybridized biochip.

### Example 4

A biochip was prepared with the same configuration as in Example 1 except that a silicon substrate was substituted for the glass substrate. Moreover, in place of the aforementioned gold or Fe fine particle colloidal modification used to label the probe DNA in Example 2, a chemical coloring (enzyme) method was performed by the ABC method using an avidin-biotin-peroxidase complex. That is, the aforementioned complex was dripped and left for 1 hour at room temperature. After washing with TBS-T (Tris Buffered Saline with added Tween 20), a tetramethylbenzene solution was dripped and left for 5 to 15 min at room temperature.

This was then washed with ultra-pure water and dried with nitrogen gas. In the results, coloring occurred when the target RNA was present but no coloring occurred when the target RNA was absent, which could be clear distinguished with the naked eye. Using the same biochip, pigment coated with avidin was also dripped in place of the aforementioned ABC method, and modification was performed for 1 to 2 hours in saturated steam at between room temperature and 37°C. After washing with TBS and drying with nitrogen gas, coloring occurred when the target RNA was present but not when the target RNA was absent, and the target specimen could be clearly detected with the naked eye.

In the above example, pigment coated with avidin was dripped after hybridization using a biochip of the same composition as in Example 2, and modification performed for 1 to 2 hours in saturated steam at between room temperature and 37°C. After washing with TBS a second label consisting of a previously dyed protein modified with biotin was dripped under the same conditions to perform modification. After washing with TBS and drying, coloring occurred more strongly than with the aforementioned dye alone when the target RNA was present while no coloring occurred when no target RNA was present, and the target RNA could be confirmed clearly with the naked eye.

Following detection by the previous described chemical colorant labeling and dye labeling, the biochips were mounted on a transport arm and set in a scanning device equipped with an electrode probe as shown in Figure 6, and voltage, current and capacitance were measured. By comparing the measurement results for voltage, current and capacitance with those for the aforementioned dry run before hybridization or label modification and the case in which label modification was performed without hybridization, it was possible to assess hybridization as electrical changes and supplement the visual detection results described above.

### Example 5

A biochip was prepared by the same methods as in Example 1. A GyrB gene (856 bp) was used as the target specimen as in Example 1. Moreover, the target specimen was modified with 50 nm silica particles, and used as a known, fixed concentration in buffer solution. The probe, a 60-nucleotide DNA which bound complementarily with the aforementioned gyrB gene, had a configuration which assumed a loop structure and was labeled in advance with magnetic fine particles.

For the biochip, electrodes a-f were prepared on the surface of a glass substrate as in Example 1, and the aforementioned probe DNA was arrayed and fixed on each electrode by the same methods as in Example 1. This was then washed with R-BFR solution and gently dried with nitrogen gas. For the probe DNA array, the concentration of buffer solution containing probe DNA was optimized with respect to the electrode surface area so that the amount fixed to each electrode would be roughly uniform.

The resulting biochip was mounted on a transport arm and set in a scanning device equipped with a magnetic head probe, and the magnetism on each electrode was measured with the results shown as "detected magnetic strength" in Table 1. "Detected magnetic strength" is shown as a relative value with respect to the value 1 of Electrode a.

Next, the biochip was hybridized with the target by the same methods as in Example 1, washed with R-BFR solution and gently dried with nitrogen gas. The biochip after hybridization was subjected to detection of scattered light by the same methods as in Example 3, to obtain the results for "detected strength of scattered light" in Table 1.

The results shown in Table 1 for strength of scattered light on each electrode after hybridization are shown relative to "detected magnetic strength" in Table 1, which is a measure of the condition of the probe on each electrode before hybridization, or in other words are shown corrected by the amount of probe as "detected strength after correction" in Table 1. That is, correcting for the amount of probe lowered the deviation in the detected value for scattered light strength considerably, from 37.4 to 6.04 after correction.

It was thus confirmed that by labeling the probe arrayed on each electrode (spot) of a prepared biochip it is possible to measure the relative amount of probe on each electrode before hybridization, and that if the relative probe amounts are known in advance the measured values for amount of target specimen forming double chains after hybridization can be accurately corrected, allowing highly accurate quantitative detection of the biochemical specimen.

**Table 1**

| Detected strength/spot | Electrode trode a | Electrode b | Electrode c | Electrode d | Electrode e | Electrode f |
|---|---|---|---|---|---|---|
| Detected magnetic strength | 1 | 1.02 | 0.97 | 1.05 | 0.95 | 0.90 |
| Detected strength of scattered light (a.u.) (s.d. = 37.4) | 800 | 810 | 780 | 830 | 763 | 725 |
| Detected strength after correction (a.u.) (s.d. = 6.04) | 800.0 | 794.1 | 804.1 | 790.5 | 803.2 | 805.6 |

### INDUSTRIAL APPLICABILITY

With the configuration of the biochip of the present invention in which a probe nucleic acid having a loop structure is arrayed on a substrate, modification with a label is performed after hybridization with a biochemical specimen in the operation of detecting a biochemical specimen, so that it is possible to label modify only the hybridized probe and only a specific site, improving accuracy in both the hybridization and label modification steps and greatly improving the accuracy of detection of the target specimen.

It is generally thought that in order to assess phenomena in vivo it is necessary to investigate the expressed amount of m-RNA rather than DNA, but because the aforementioned probe of the present invention which employs a loop structure is highly sensitive with little background noise, and because there is no need to label modify the specimen, the movement of m-RNA can be detected directly without preparing c-DNA.

Moreover, because DNA-RNA binding is more stable than DNA-DNA binding, using probe RNA rather than DNA to detect DNA offers the advantage of greater detection sensitivity and allows stable detection accuracy to be maintained.

In addition to offering greater accuracy than conventional methods in hybridization of the target probe nucleic acid with the biochemical specimen and label modification itself, the biochip of the present invention allows these to be assessed electrically, electromagnetically, electro-optically or electro-magneto-optically as shown in the examples, so that highly accurate detection of biochemical specimens and quantitative detection can be achieved.

## Claims

1. A method for detecting a biochemical reactant comprising the steps of:
hybridizing a biochemical specimen with a probe nucleic acid on a biochip wherein the probe nucleic acid has a loop structure and is arrayed on one or two or more electrodes provided on a surface of a substrate or a substrate analog and wherein the probe nucleic acid is optionally pre-labelled; and
detecting/discriminating a complex of the probe nucleic acid with the biochemical specimen by means of at least one of an electrical, magnetic or optical change on the surface of the biochip.

2. A method for detecting a biochemical reactant comprising the steps of:
hybridizing a biochemical specimen with a probe nucleic acid on a biochip wherein the probe nucleic acid has a loop structure and is arrayed on one or two or more electrodes provided on a surface of a substrate or a substrate analog wherein the probe nucleic acid is optionally pre-labelled; labelling during and/or after hybridization one or both of the biochemical specimen and the probe nucleic acid; and
detecting/discriminating a complex of the probe nucleic acid with the biochemical specimen by means of at least one of an electrical, magnetic or optical change on the surface of the biochip.

3. A method for detecting a biochemical reactant comprising the steps of:
hybridizing a pre-labelled biochemical specimen with a probe nucleic acid on a biochip wherein the probe nucleic acid has a loop structure and is arrayed on one or two or more electrodes provided on a surface of a substrate or a substrate analog and wherein the probe nucleic acid is optionally pre-labelled; and
detecting/discriminating a complex of the probe nucleic acid with the biochemical specimen by means of at least one of an electrical, magnetic or optical change on the surface of the biochip.

4. A method for detecting a biochemical reactant comprising the steps of
hybridizing a pre-labelled biochemical specimen with a probe nucleic acid on a biochip wherein the probe nucleic acid has a loop structure and is arrayed on one or two or more electrodes provided on a surface of a substrate or a substrate analog and wherein the probe nucleic acid is optionally pre-labelled;
labelling during and/or after hybridization one or both of the biochemical specimen and the probe nucleic acid; and
detecting/discriminating a complex of the probe nucleic acid with the biochemical specimen by means of at least one of an electrical, magnetic or optical change on the surface of the biochip.

5. A method for detecting a biochemical reactant according to any one of Claims 1 to 4, wherein in the detection/discrimination step a measurement result obtained from a measurement to assess at least one of the electrical, magnetic and optical changes to the surface of the biochip before the hybridization operation is used as the standard for comparing the measurement results for the biochip following each step.

6. A method for detecting a biochemical reactant according to any one of Claims 1 to 4, wherein in the detection/discrimination step measurements are performed before and after the hybridization step and/or before and after the labelling step to assess at least one of the electrical, magnetic and optical changes to the surface of the biochip, and these results are compared.

7. A method for detecting a biochemical reactant according to any one of Claims 1 to 4, wherein in the detection/discrimination step measurements are performed before the hybridization operation to assess at least one of the electrical, magnetic and optical changes to the surface of a biochip having a plurality of electrodes, and the relative amounts of probe nucleic acids on each electrode are calculated in advance and used as a correction reference for the measurement values after each step.

8. A method for detecting a biochemical reactant according to any of the Claims 1 to 4, wherein the pre-labelled probe nucleic acid or biochemical specimen is labelled in a multi-stage modification having two or three or more stages in which a second label is added targeting a previously attached first label.

9. A method for detecting a biochemical reactant according to any one of Claims 1 to 4, wherein the method of labelling the probe nucleic acid or biochemical specimen is a multi-stage modification in two or three or more stages in which modification with a first label is followed by modification with a second label targeting the first label.

10. A method for detecting a biochemical reactant according to any one of Claims 1 to 4, wherein the label is a fine metal particle (including Si), a magnetic particle, a ceramic fine particle, a fluorescent label, a fluorescent dye, a dye, a chemical colorant and/or a semiconductor.

11. A method for detecting a biochemical reactant according to any one of Claims 1 to 4, wherein the method of detecting/discriminating electrical changes on the surface of a biochip is a method of detecting/discriminating at least a change in a current value, voltage value or resistance value on a biochip or electrode, or a change in capacitance on the surface of a biochip.

12. A method for detecting a biochemical reactant according to any one of Claims 1 to 4, wherein the method of detecting/discriminating an electrical and/or magnetic change on the surface of a biochip comprises the steps of:
detecting/discriminating at least a change in a current value, voltage value or resistance value on a biochip or electrode, or a change in capacitance on the surface of a biochip; and/or
magnetically detecting/discriminating a signal from a complex forming a double chain.

13. A method for detecting a biochemical reactant according to any one of Claims 1 to 4, wherein the method of detecting/discriminating an electrical and/or optical change on the surface of a biochip comprises the steps of:
detecting/discriminating at least a change in a current value, voltage value or resistance value on a biochip or electrode or a change in capacitance on the surface of a biochip; and
optically detecting/discriminating a signal from a complex forming a double chain.

14. A method for detecting a biochemical reactant according to any one of Claims 1 to 4, wherein the method of detecting/discriminating an electrical, magnetic and/or optical changes on the surface of a biochip comprises the steps of:
detecting/discriminating at least a change in a current value, voltage value or resistance value on a biochip or electrode, or a change in capacitance on the surface of a biochip; and
magnetically and optically detecting/discriminating a signal from a complex forming a double chain.

15. A biochip constituted by a substrate or analog thereof which has at least one electrode formed on its surface and having a probe nucleic acid arrayed on the surface of said electrode, wherein the probe nucleic acid has a loop structure.

16. A biochip constituted by a substrate or analog thereof which has at least one electrode formed on its surface and has a probe nucleic acid arrayed on the surface of said electrode, wherein the arrayed probe nucleic acid has a loop structure in which the principal part which binds complementarily with the biochemical specimen is located on the substrate or substrate analog side.

17. A biochip constituted by a substrate or analog thereof which has at least one electrode formed on its surface and has a probe nucleic acid arrayed on the surface of said electrode, wherein the arrayed probe nucleic acid has a loop structure in which the free end not fixed to the surface of the electrode or the site thereof capable of being modified with a label is located on the substrate or substrate analog side.

18. A biochip constituted by a substrate or analog thereof which has at least one electrode formed on its surface and has a probe nucleic acid arrayed on the surface of said electrode, wherein the arrayed probe nucleic acid has a loop structure in which a site modified with a first label which allows further optional modification with a second label is located on the substrate or substrate analog side.

19. A biochip constituted by a substrate or analog thereof which has at least one electrode formed on its surface and has a probe nucleic acid arrayed on the surface of said electrode, wherein the arrayed probe nucleic acid has a loop structure in which a previously attached label is located on the substrate or substrate analog side.

20. A biochip according to Claim 18 or Claim 19, wherein the label is selected from a metal fine particle (including Si), magnetic particle, ceramic fine particle, fluorescent label, fluorescent dye, dye, chemical colorant and a semiconductor.

21. A biochip according to any one of Claims 15 to 19, wherein the substrate or substrate analog material is glass or semiconductor silicon.

22. A biochip according to any one of Claims 15 to 19, wherein the substrate or analog thereof is capable of being embedded in a separately prepared electrical circuit board.
